# EUROPEAN PATENT SPECIFICATION

(11) **EP 2 538 870 B1**
(45) Date of publication and mention of the grant of the patent: **01.01.2020**
(21) Application number: 11747841.2
(22) Date of filing: 02.02.2011
(51) Int. Cl.: A61K 6/00, A61K 6/027, A61K 6/06

(54) **GLASS AND/OR GLASS CERAMIC PARTICLES CONTAINING COMPOSITION FOR APPLICATION ON A DENTAL ARTICLE, PROCESS AND USE THEREOF**
GLAS- UND/ODER GLASKERAMIKPARTIKEL MIT EINER ZUSAMMENSETZUNG ZUR ANWENDUNG AUF EINEM ZAHNÄRZTLICHEN ARTIKEL SOWIE VERFAHREN DAFÜR UND VERWENDUNG DAVON
COMPOSITION CONTENANT DES PARTICULES DE VERRE ET/OU VITROCÉRAMIQUES POUR APPLICATION SUR ARTICLE DENTAIRE, SON PROCÉDÉ ET SON UTILISATION

(30) Priority: 25.02.2010 EP 10154606
(43) Date of publication of application: 02.01.2013
(73) Proprietor: 3M Innovative Properties Company, St. Paul, MN 55133-3427 (US)
(72) Inventor: SCHECHNER, Gallus, D-82229 Seefeld (DE); JAHNS, Michael, D-82229 Seefeld (DE)
(74) Representative: Brem, Roland
(86) International application number: PCT/US2011/023422
(87) International publication number: WO 2011/106132

(56) References cited:
- EP-A1- 2 156 817
- US-A- 5 028 638
- US-A- 5 433 956
- US-A- 5 874 101

## Description

### Field of the Invention

The invention relates to a process for producing a dental restoration or part thereof comprising providing a container comprising a composition to be used for application on a dental article. The invention also relates to the use of the composition for producing dental restorations or parts thereof.

### Background Art

Dental restorations or prostheses are often made of two or more components, with the individual components providing different characteristics for the restoration. For example, a support structure or frame may provide excellent structural support, and a facing may provide excellent aesthetics. The frame generally is a supporting structure for the dental restoration that provides mechanical stability and usually comprises an interface by which the restoration can be affixed to a prepared tooth of a patient. The facing typically provides for pleasing aesthetic characteristics that gives the restoration the shape and appearance of natural teeth. In addition, both the frame and the facing are shaped to fit well with the adjacent and opposed teeth in a patient's mouth.

In the recent years ceramic materials have been widely used for making high-quality dental restorations because of their good physical, aesthetic and biological properties. These restorations are often manufactured by an automated process, which typically includes:
- capturing data representing the shape of a patient's teeth, for example by scanning a plaster model of the patient's teeth or alternatively by scanning the actual teeth in the patient's mouth;
- designing the shape of a frame based on the captured data using software, such as computer-aided design (CAD) software; and
- manufacturing the frame to correspond to the designed shape, for example, by an automated Computer Numerical Controlled (CNC) machine.

There are approaches to integrating the steps of capturing, designing and manufacturing in a Computer Integrated Manufacturing (CIM) system. An exemplary CIM system for making frames for dental restorations is available from 3M ESPE AG (Seefeld, Germany) under the trade designation LAVA™.

Although such CIM systems would allow the manufacture of an integrated dental restoration (frame and facing together, in the example mentioned above), it is difficult to provide a single ceramic material that provides both the necessary structural durability and good aesthetics. Therefore the CIM system is normally used to manufacture the frame from a ceramic material that provides the required mechanical durability without regard to its aesthetic properties, after which a final layer or facing is applied to the frame to provide the necessary aesthetic properties. A facing of this type is very often manually prepared by skilled dental technicians, for example by manually applying several layers of a polymeric material or a glass-ceramic material, to provide the appropriate color, translucency, and other properties.

Another common method for manually preparing a facing is the "press over" technique. A frame is manufactured as described above, and manually covered with a wax layer or "wax-up" with an outer surface that corresponds to the desired final shape of the tooth. That wax-up is used to form a pattern for a mold. The mold is then heated in a furnace so that the wax is burned off, and the frame remains as a core in the mold. The space between the core and the interior of the mold is then filled with a molten glass-ceramic material that is, for example, obtained from melting a ceramic pellet in channel or previously molten and poured into the channel, where it flows around the frame and fuses with the frame to form the facing. The restoration may then be removed from the mold, polished as necessary, and provided to the dentist for application to the patient's tooth.

According to a more recent approach, the dental facing can also be milled out of blocks and adhesively fixed on the dental support structure.

WO 2006/120255 A2 relates to a method for production of a tooth replacement piece using CAD/CAM technology, wherein the tooth replacement piece comprises a skeleton as first component and a facing piece as a second component. Fixing of the first component to the second component can done by using a fixing composition comprising a low melting glass material or organic adhesive.

To adjust and to keep the distance between the support structure and the facing structure it is proposed to mount a distance piece to the inner side of the facing piece.

US 5,346,397 describes a process for making artificial porcelain teeth comprising a joining step of a skeleton part and a facing piece with the help of small spheres of defined diameters in order to obtain a defined spacing between skeleton part and facing part. The small spheres are added into the gap between skeleton and facing so that their diameter rules the distance between them.

US 6,371,762 B1 refers to a ceramic tooth restoration and method for manufacturing. The tooth restoration comprises two neighbouring tooth replacement elements, a connecting member and a glass paste for sintering said connecting members.

In DE 10 347 900 a pasty or gel-like mass used in dentistry is described. The paste is used for fitting caps, crowns and bridges and is arranged in a tube or cartridge and/or injection packing with an opening.

WO 2008/144342 A2 describes a method for making a facing for a dental restoration. It is described that a facing precursor can be assembled to a frame using a slurry. The slurry used according to a specific example can comprises a glass ceramic powder material and a liquid comprising a certain amount of polyglykol 4000, a certain amount of propanediol and a certain amount of water.

US 5,874,101 (Zhong et al.) describes a process for making bioactive glasses by preparing a reaction mixture of reactants capable of forming a sol-gel, aging the reaction mixture, near equilibrium drying a gel resulting from the reaction mixture, and heating the near equilibrium-dried gel.

US 5,433,956 (Patel) relates to glassy coatings made by curing in situ a coating of a sol gel of tetraethoxysilicon, water and alcohol, filled with kaolinite or with flat plates of mica, which may be colored by Fe₂O₃/TiO₂ coatings.

Commercially available modelling liquids and powders typically have to be mixed before use and are not available as a ready-to-use product.

Simply mixing both components in order to provide a ready-to-use product, however, does not always work. The obtained mixture is often not sufficiently storage stable.

Adding only an organic surfactant to the composition might help to partially solve this problem.

Examples of such compositions are so-called opaquer pastes, which are typically stabilized by adding a huge amount of glycerol. Nevertheless, mechanical stirring or shaking of the composition before use is still required. Moreover, it has been observed that the shelf life of these pastes is rather limited and closed vessels, which do not allow for an easy re-dispersion of the components, might have to be disposed of after about 1 year. Another drawback of organic stabilizers is the fact that the organic material has to be somehow removed from the slurry after application of the slurry on a surface in order to avoid detrimental effects on the final product to be produced.

Currently there is no proper way to stabilize a slurry of glass particles over a long period of time without applying mechanical stirring. Because of this, it is common practice to deliver the glass powder as a stand-alone product and the slurry is produced by the customer prior to use.

Thus, there is still room for improvement especially with regard to the requirements to be fulfilled with respect to modern dental materials.

### Summary

More particularly, a composition for application on a dental article is desired which is storage-stable, that is, which can be stored for a sufficient period of time without significant agglomeration or separation of the individual components being present in the composition.

The invention is directed to an ex vivo process for producing a dental restoration or part thereof comprising the steps of providing a container and applying the composition contained or stored in the container on the surface of a dental article as described in the claims. Described is also a kit of parts comprising a container containing the composition as described in the present text and an application device.

In another embodiment, the invention relates to a process as described in the claims comprising the following steps:
a) providing a dental facing precursor and a dental support structure, both having an upper and a lower surface,
b) attaching the dental facing precursor with its lower surface to the upper surface of the dental support structure, comprising the step of placing the composition contained or stored in a container as described the present text between the lower surface of the dental facing precursor and the upper surface of the dental support structure.

A further aspect of the invention is directed to a kit of parts as described in the claims comprising at least two containers as described in the present text, wherein at least one of the compositions contained or stored in the container comprises in addition a colorant.

### Brief Description of Drawings

Fig. 1 shows an example of a process for producing the composition.
Fig. 2 shows an example of a container, which can be used for storing the composition.
Fig. 3 shows an example of another container, which can be used for storing the composition.

It has been found that the composition contained or stored in a container described in the present text fulfils the practitioners' needs especially with regard to storage stability.

The composition can be stored for a sufficiently long period of time without showing aggregation, separation or deposition of the components contained therein. A sufficiently long period of time includes a time frame of at least 10 days under ambient conditions (e.g. 23°C). A typical shelf life for the composition is from 1 month to 2 years.

It has been found that the use of a crosslinkable binder precursor usually prevents or at least significantly reduces or slows down the sedimentation or agglomeration tendency of the glass and/or glass ceramic particles having the result that an essentially homogeneous distribution of the particles in the slurry-like composition for a sufficient period of time without the need for stirring can be ensured.

By adding a crosslinkable binder precursor, the slurry-like composition typically turns into a non-flowing composition which can be returned to its former flowing state if desired e.g. by applying shear forces. A simple means for achieving this is the application of the slurry through a nozzle.

Thus, a "ready-to-use" composition can be produced and stored for a sufficient long period of time. The composition can be provided to the customer (e.g. a dental technician) in optimized condition for use (i.e. adjusted composition for specific applications) compared to a set of components which otherwise have to be mixed by hand before use. Since the composition is contained in a sealable container, it can be assured that the content of the composition does not severely change over time, e.g. due to evaporation of the liquid contained in the composition. Thus, the composition can always be provided in a sufficient quality.

The composition typically also has beneficial rheological properties.

The composition can be applied in sufficient thin layers and shows a beneficial flowing behaviour. The ability of creating thin layers might help to avoid unwanted bite enlargements when attaching a dental facing to a dental support structure.

Moreover, the composition has a sufficient wetting behaviour especially with respect to articles comprising or essentially consisting of ceramic materials such as YTZP ceramic (Yttrium stabilized zirconia).

The composition is typically also sufficiently viscous and sticky, thus, allowing the practitioner to shape and model the composition, if desired. Shaping or modelling of the composition might be desirable, if after the process of attaching a dental facing to a dental support structure it is realized, that at the edge or border of the articles to be attached or to be mated some mating material is missing. Thus, the composition provides for the ability to correct or modify the mating layer.

Moreover, the composition can easily be modelled and does typically better adhere to the material a dental restoration is made of than to the application instrument (e.g. brush).

Compositions showing a decrease in viscosity at a constant shear rate were found to be beneficial, as well.

It was found that the composition is particularly useful for attaching dental facing precursors comprising glass and/or glass ceramic materials to dental support structures comprising zirconia. Due to its open-celled structure dental facing precursors have a different and beneficial wetting behaviour and typically a different absorbing behaviour compared to dental facings having a closed-celled structure or having already been sintered to final density. The wetting behaviour can be analyzed by measuring the contact angle of a liquid drop on a surface.

Moreover, when using the composition for attaching dental facings to dental support structures dental restorations can be obtained showing essentially no gaps, bubbles or defects at the interface between the dental support structure and the dental facing after conducting a sintering step. This can be proven by analyzing the interface (cross section) of two materials attached to each other.

It has also been found that the composition can be combusted without leaving residues which might be detrimental to the performance of the resulting dental restoration. This typically facilitates a later sintering step of the composition or a product to which the composition has been applied.

Except for the glass and/or glass ceramic material which might be present, all other components in the composition have either a boiling point far below the sintering temperature or can be burnt essentially completely.

This is mainly caused by the fact that the composition does typically not require the addition of an organic binder.

In contrast to organic binder materials, the crosslinkable binder precursor used in the present invention does typically not negatively influence the overall performance of the final product, e.g. a dental restoration.

### Definitions

Unless otherwise specified, within the context of the text of the invention, the following terms have the following meanings.

The term "dental article" means any article which can be used in the dental field, especially dental restorations, tooth models and parts thereof. Dental restorations are typically comprised of at least two parts: a dental framework (frame) and a dental veneer (facing). Examples include crowns, bridges. The term "dental article", however, also includes inlays, onlays, facings, abutments and implants. The surface of a tooth is considered not to be a dental article.

The term "dental facing" within the meaning of this invention refers to the aesthetic part of a dental restoration, meaning the part comprising an outer surface of the finished restoration. The dental facing is further adapted to be applied to a frame or dental support structure which forms another part of the dental restoration, and the dental restoration is in turn applied to a tooth. The dental facing is preferably arranged at those parts of the dental support structure that are likely to be visible in a patient's mouth, or that in particular functionally co-operate with the adjacent or opposed teeth of a patient, for example. A "dental facing precursor" refers to the dental facing in a pre-stage, that is, to a stage, where the dental facing is not finished yet. A dental facing precursor has a 3-dim. shape and is made of or comprises a certain material. The shape of the dental facing precursor corresponds essentially to the shape of the finished dental facing, however, is typically enlarged at least with respect to the outer, visible surface.

A dental framework or a dental veneer usually has a 3-dimensional inner and outer surface including convex and concave structures. The outer surface of the dental framework typically corresponds essentially to the inner surface of the dental veneer. The inner surface of the dental framework typically corresponds essentially to outer surface of a prepared tooth stump, whereas the outer surface of the dental veneer typically corresponds essentially to the final dental restoration.

Dental ceramic frameworks are typically made of or comprise oxide ceramic materials including ZrO₂ or Al₂O₃. Compared to other framework such as pottery or paving stones, the dental framework is small and filigree and of high strength. The thickness of the dental framework can vary from very thin, e.g. at the edges and rims (below about 0.1 mm) to considerably thick, e.g. in the biting area (up to about 7 mm). However, dental frameworks may also be made of or comprise metal or metal alloys.

Dental veneers are also small and filigree objects. The strength of dental veneers, however, is typically less compared to dental frameworks. Dental veneers are typically made of or comprise glass or glass ceramic materials.

A "porous material" refers to a material comprising a partial volume that is formed by voids, pores, or cells in the technical field of ceramics. Accordingly an "open-celled" structure of a material sometimes is referred to as "open-porous" structure, and a "closed-celled" material structure sometimes is referred to as a "closed- porous" structure. It may also be found that instead of the term "cell" sometimes "pore" is used in this technical field. The material structure categories "open-celled" and "closed-celled" can be determined for different porosities measured at different material samples (e.g. using a mercury "Poremaster 60-GT" from Quantachrome Inc., USA) according to DIN 66133. A material having an open-celled or open-porous structure can be passed through by e.g. gases.

"Glass" means an inorganic non-metallic amorphous material which is thermodynamically an undercooled and frozen melt. Glass refers to a hard, brittle, transparent solid. Typical examples include soda-lime glass and borosilicate glass. A glass is an inorganic product of fusion which has been cooled to a rigid condition without crystallizing. Most glasses contain silica as their main component and a certain amount of glass former

"Glass-ceramic" means an inorganic non-metallic material where one or more crystalline phases are surrounded by a glassy phase so that the material comprises a glass material and a ceramic material in a combination or mixture. Thus, a glass ceramic is a material sharing many properties with both glass and more traditional crystalline ceramics. It is formed as a glass, and then made to crystallize partly by heat treatment. Unlike sintered ceramics, glass-ceramics have no pores between crystals. Instead, the space between the crystallites is filled by the glassy matrix. Glass ceramics mainly refer to a mixture of lithium-, silicon-, and aluminium-oxides.

"Ceramic" means an inorganic non-metallic material that is produced by application of heat. Ceramics are usually hard, porous and brittle and, in contrast to glasses or glass ceramics, display an essentially purely crystalline structure.

The term "proportionally enlarged" means that each of the three dimensions of an enlarged object is enlarged relative to the corresponding dimension of the original object by preferably substantially the same magnification factor. Further, "proportionally enlarged" may include tolerances of the magnification factor in each dimension so that each of the three dimensions of the enlarged object may be enlarged relative to the corresponding dimension of the original object by three individual magnification factors with at least two of the individual magnification factors being different from each other by 1% to 5%.

The term "proportionally reduced" means that each of the three dimensions of a shrunken object is reduced relative to the corresponding dimension of the original object by preferably substantially the same shrinkage factor. Further, "proportionally reduced" may include tolerances of the shrinkage factor in each dimension so that each of the three dimensions of the shrunken object may be reduced relative to the corresponding dimension of the original object by three individual shrinkage factors with at least two of the individual shrinkage factors being different from each other by 1% to 5%.

By "machining" is meant milling, grinding, cutting, carving, or shaping a material by a machine. Milling is usually faster and more cost effective than grinding. A "machinable article" is an article having a 3-dimensional shape and having sufficient strength to be machined.

"Sintering" means making objects from a powder, by heating the material (typically below its melting point - solid state sintering) until its particles adhere to each other.

A "powder" means a dry, bulk solid composed of a large number of very fine particles that may flow freely when shaken or tilted.

"Density" means the ratio of mass to volume of an object. The unit of density is typically g/cm³. The density of an object can be calculated e.g. by determining its volume (e.g. by calculation or applying the Archimedes principle or method) and measuring its mass.

A "liquid" within the meaning of the invention is any solvent or liquid which is able to at least partially disperse or dissolve the crosslinkable binder precursor at ambient conditions (e.g. 23°C).

A "particle" means a substance being a solid having a shape which can be geometrically determined. The shape can be regular or irregular. Particles can typically be analysed with respect to e.g. grain size and grain size distribution.

A dental ceramic article or framework is classified as "presintered" within the meaning of the invention if the dental ceramic framework has been treated with heat (temperature range from 900 to 1100°C) for 1 to 3 hours to such an extend that the raw breaking resistance (Weibull strength Sigma 0) of the dental ceramic article or framework is within a range of 15 to 55 MPa or 30 to 50 MPa (measured according to the "punch on three ball test" (biaxial flexural strength) described in DIN EN ISO 6872, edition March 1999, with the following modifications: diameter of steel ball: 6 mm; diameter of support circle: 14 mm; diameter of flat punch: 3.6 mm; diameter of sample disc: 25 mm, thickness of sample disc: 2 mm; no grinding and polishing of samples.).

A presintered dental ceramic article or framework typically has a porous structure and its density (usually 3.0 g/cm³ for an Yttrium stabilized ZrO₂ ceramic) is less compared to a completely sintered dental ceramic framework (usually 6.1 g/cm³ for an Yttrium stabilized ZrO₂ ceramic). The diameter of the pores can be in a range of 50 nm to 150 nm (corresponding to 500 to 1500 Ǻ). A typical pore diameter is 120 nm.

Presintering of a glass or glass ceramic material is typically effected in a temperature range of 500 to 750 °C.

The terms "sintering" or "firing" are used interchangeably. A pre-sintered ceramic framework shrinks during a sintering step, that is, if an adequate temperature is applied. The sintering temperature to be applied depends on the ceramic material chosen. For ZrO₂ based ceramics a typical sintering temperature range is 1200 °C to 1500 °C. Al₂O₃ based ceramics are typically sintered in a temperature range of 1300 °C to 1700 °C. Glass ceramic materials are typically sintered in a range of about 700 to 1100 °C for 1 to 3 hours.

Sintering typically includes the densification of a porous material to a less porous material (or a material having less cells) having a higher density, in some cases sintering may also include changes of the material phase composition (for example, a partial conversion of an amorphous phase toward a crystalline phase). A porous material sometimes refers to a material comprising a partial volume that is formed by voids, pores, or cells in the technical field of ceramics. Accordingly an "open-celled" structure of a material sometimes is referred to as "open-porous" structure, and a "closed-celled" material structure sometimes is referred to as a "closed-porous" structure. It may also be found that instead of the term "cell" sometimes "pore" is used in this technical field.

The structure of a material as referred to in this specification may be categorized as "open-celled", "closed-celled" and "generally free of cells".

The term "open-celled" relates to an "open porosity" according to the mercury porosimetry as defined in DIN 66133. Typical values are between 6% and 35%, of between 15% and 35%, or between 30% and 35%.

The term "closed-celled" relates to a "closed porosity". Closed cells are those cells which are not accessible from the outside and cannot be infiltrated by gases under ambient conditions.

The unit "cells per mm²" is related to the number of cells present on a cross section of the sample to be analysed. A suitable test method is given in DIN 13925.

The volume of a sample can be determined based on the overall outer dimensions of the sample. The density of the sample can be calculated from the measured sample volume and the sample mass. The total volume of glass ceramic material can be calculated from the mass of the sample and the density of the used material.

The total volume of cells in the sample was assumed to be the remainder of the sample volume (100% minus the total volume of material).

A dental ceramic framework is classified as "absorbent" if the dental ceramic framework is able to absorb a certain amount of a liquid, comparable to a sponge. The amount of liquid which can be absorbed depends e.g. on the chemical nature of the dental ceramic framework, the viscosity of the solvent, the porosity and pore volume of the dental ceramic framework. E.g. a pre-sintered dental ceramic article, that is an article which has not been sintered to full density, is able to absorb a certain amount of liquid. Absorbing of liquids is typically only possible if the article has an open-porous structure.

"Sintering" means making objects from a powder, by heating the material (typically below its melting point - solid state sintering) until its particles adhere to each other.

A "sol/gel reaction" is a wet-chemical technique (sometimes also referred to as "Chemical Solution Deposition") for the fabrication of materials starting either from a chemical solution or colloidal particles (e.g. nanoscale particle) to produce an integrated network (gel). Typical precursors are metal alkoxides and metal chlorides, which undergo hydrolysis and polycondensation reactions to form a colloid, a system composed of solid particles (size ranging from 1 nm to 1 µm) dispersed in a solvent. The sol evolves then towards the formation of an inorganic continuous network containing a liquid phase (gel). Formation of a metal oxide involves connecting the metal centers with oxo (M-O-M) or hydroxo (M-OH-M) bridges, therefore generating metal-oxo or metal-hydroxo polymers in solution. The drying process serves to remove the liquid phase from the gel thus forming a porous material. Afterwards, a thermal treatment (firing) may be performed in order to favor further polycondensation and enhance mechanical properties. From a chemical standpoint of view, sol/gel reactions are well known to a person skilled in the art. Sol/gel reactions are described in more detail e.g. in "Sol-Gel-Science: The Physics and Chemistry of Sol-Gel Processing" from C. Jeffery Brinker and George W. Scherer (ISBN: 0-12-134970-5).

A "crosslinkable binder precursor" is able to undergo a sol/gel reaction. That is, the binder precursor can undergo a hydrolysis and/or a polycondensation reaction, thereby forming a sol or colloid. This network ultimately binds the solid particles contained in the composition.

A "colloid" is described as a system comprising particles (e.g. having a size ranging from 1 nm to 1 µm) dispersed in a solvent or liquid

A "hardenable compound" is any compound which can be cured or solidified e.g. by chemical crosslinking through radiation-induced polymerization, crosslinking by using an initiator or heating.

An "initiator" is a substance being able to start the hardening process of a hardenable compound.

The terms "curing", "hardening" or "condensing" are used interchangeable throughout the text and mean the reaction of components with each other thereby creating a network.

A composition or solution is "essentially or substantially free of' a certain component within the meaning of the invention, if the composition or solution does not contain said component as an essential feature. Thus, said component is not wilfully added to the composition or solution either as such or in combination with other components or ingredient of other components. Ideally the composition or solution does not contain the said component at all. However, sometimes the presence of a small amount of the said component is not avoidable e.g. due to impurities contained in the raw materials used.

"Ambient conditions" mean the conditions which the inventive solution is usually subjected to during storage and handling. Ambient conditions may, for example, be a pressure of 900 to 1100 mbar, a temperature of -10 to 60 °C and a relative humidity of 10 to 100 %. In the laboratory ambient conditions are adjusted to 20 to 25 °C and 1000 to 1025 mbar.

As used herein, "a", "an", "the", "at least one" and "one or more" are used interchangeably. The terms "comprises" or "contains" and variations thereof do not have a limiting meaning where these terms appear in the description and claims. Also herein, the recitations of numerical ranges by endpoints include all numbers subsumed within that range (e.g., 1 to 5 includes 1, 1.5, 2, 2.75, 3, 3.80, 4, 5, etc.).

### Detailed Description

The composition without glass and/or glass ceramic material and without a crosslinkable binder precursor or with a precursor before starting the polymerisation reaction can be characterized by one or more of the following features:
- Viscosity of composition without glass and/or glass ceramic material, that is the composition, which besides the glass and/or glass ceramic material, contains all the other components: being in the range of 10 mPas to 1,000 mPas or being in the range of 20 mPas to 500 mPas, or being in the range of 30 mPas to 100 mPas measured at 23°C with a shear rate of 50 s⁻¹ (measured with a viscosimeter MCR301 (Anton Paar Comp.), cone plate geometry, diameter 25mm, temperature of 23°C, shear rate s d(gamma)/dt of 50 s⁻¹).

The viscosity measurement can be done as follows: A viscosimeter MCR301 (from Anton Paar Comp.) can be used. A portion of the liquid composition is placed between two steel discs with a diameter of 8 mm and a gap of 1 mm at a temperature of 23°C. The gap is filled completely with the liquid/powder composition. Excess liquid/powder composition is removed. To avoid an undesired drying of the slurry during the measurement a ribbon of wetted paper tissue is laid around the discs in order to raise the humidity. The shear rate between the rotating discs d(gamma)/dt is set constantly to 50 s⁻¹. The measurement is done 500 s after starting the shearing process of the composition.
- The composition can be combusted without leaving organic residues, if heated up to a temperature of about 750 °C for about 1 min at ambient pressure (e.g. 1013 mbar).
- The content of organic components (containing carbon) is below 7 wt.-% or below 3 wt.-% or below 2 wt.-%.

The term "can be combusted without leaving residues" means that if about 200 mg of the composition is heated up to a temperature of about 750 °C for about 1 min at ambient pressure, no visible (visible with the human eye) deposits can be found. That is, the composition either evaporates or can be burnt producing only gaseous components including carbon oxide and water. Except for the glass and/or glass ceramic material being present in the composition essentially no further components are left. This can be determined, if desired, e.g. by visually (with the human eye only) inspecting a final dental restoration obtained after a firing step. A greyish appearance of the dental restoration can be an indicator for a dental composition not fulfilling the above mentioned feature. E.g., using a glycerol containing composition typically leads to a dental restoration having a greyish appearance, something which is not desirable.

A low content of organic materials may facilitate the full combustion of the organic material during a heating or firing step conducted during the production of a dental restoration.

The viscosity of the complete composition (including glass and/or glass ceramic material) is typically within a range of 500 to 20,000 mPas, or 1,000 to 10,000 mPas or 1,500 to 5000 mPas.

The composition comprises a liquid.

The nature and structure of the liquid to be used in the process described in the present invention is not particularly limited, unless the intended purpose cannot be achieved.

Suitable liquids can be characterized by at least one of the following features:
- boiling point: 60 to 120 °C,
- freezing point: -120 to 0 °C,
- density: 0.7 to 1.2 g/cm3,
- mixable with water,
- neutral pH-value (pH about 7), if mixed with water.

It can be preferred, if the liquid used has similar properties as the by- or condensation products produced during the hardening process or condensing step of the inorganic binder. For example, if during the hardening step a solvent like methanol is produced, the liquid should be miscible with methanol as it is the case for water and alcohols.

Specific examples of liquids which can be used include water, alcohols (including methanol, ethanol n- and iso-propanol), ketons (including acetone) and mixtures or combinations thereof.

Water which can be used in the inventive composition includes de-ionized water and is sometimes preferred.

The content of liquid in the composition is not particularly limited, unless the desired thin layers cannot be obtained.

The composition may comprise further liquids.

The liquid can be present in an amount ranging from 15 wt.-% to 60 wt.-%, or from 20 wt.-% to 40 wt.-% or from 25 wt.-% to 35 wt.-%, with respect to the whole composition or mixture, respectively.

The liquid can be present in an amount of at least 15 wt.-% or at least 20 wt.-% or at least 25 wt.-% with respect to the whole composition or mixture, respectively.

The liquid can be present in an amount up to 35 wt.-% or up to 40 wt.-% or up to 60 wt.-% with respect to the whole composition or mixture, respectively.

The composition comprises a glass and/or glass ceramic material.

The nature and structure of the glass and/or glass ceramic material is not particularly limited unless it is detrimental to the desired performance of the composition.

The glass or glass ceramic powder may consist essentially of, or consist only of a glass or glass ceramic material. The glass or glass ceramic material is preferably selected to be compatible for use in human bodies. Furthermore, the glass or glass ceramic material is preferably selected to provide good aesthetic appearance for the dental restoration, in particular when combined with a dental framework.

The grain size of the particles of the material should be sufficiently low to facilitate the provision of a homogeneous slurry. The glass and/or glass ceramic material which can be used is typically in a powder form. The mean or average grain size of the particles of the material or powder can be e.g. in the range from 1 µm to 150 µm or 5 to 50 µm. During the production process, the glass or glass ceramic material is usually subjected to a grinding process and thus typically comprises particles of random shape. Geometric particle shapes like perfect spheres or platelets are usually not found in the material. Thus, the particles are typically not plated.

Glass or glass ceramics materials which can be used can be characterized by at least one of the following features:
- comprising: 55 wt.-% to 75 wt.-% of silicon oxide, 8 wt.-% to 22 wt.-% of aluminum oxide, 0 wt.-% to 8 wt.-% of boron oxide, 3 wt.-% to 12 wt.-% of potassium oxide, 4 wt.-% to 12 wt.-% of sodium oxide, 0 wt.-% to 2 wt.-% of cerium oxide, 0 wt.-% to 2 wt.-% of tin oxide, 0 wt.-% to 3 wt.-% of zinc oxide, 0 wt.-% to 4 wt.-% of phosphor oxide, 0 wt.-% to 3 wt.-% of calcium oxide, 0 wt.-% to 3 wt.-% of lithium oxide, and 0 wt.-% to 1 wt.-% of fluoride, 0 wt.-% to 3 wt.% of lanthanum oxide or lanthanide oxide.
- Coefficient of thermal expansion: 8 ^{∗} 10⁻⁶K⁻¹ to 15.8 ^{∗} 10⁻⁶K⁻¹ or 8 ^{∗} 10⁻⁶K⁻¹ to 9 ^{∗} 10⁻⁶K⁻¹ or 12 ^{∗} 10⁻⁶K⁻¹ to 13.6 ^{∗} 10⁻⁶K⁻¹ or from 15 ^{∗} 10⁻⁶K⁻¹ to 15.8 ^{∗} 10⁻⁶K⁻¹.
- mean particle size: range from 1 µm to 60 µm, or from 5 to 40 µm (measured with laser diffraction);
- melting temperature (range): around or less than about 1000 °C
- density: 2.0 to 2.8 or 2.2 to 2.6 g/cm³ (according to the technical data sheet provided by the manufacturer) and/or
- glass transition temperature: 500 to 600 °C or 520 to 580 °C, preferably about 550 °C.

If larger glass or glass ceramic particles are used (e.g. particles having an average particle diameter above 1 µm or above 2 µm or above 5 µm), the sedimentation process sometimes proceed faster than the gelation reaction of the crosslinkable binder precursor.

This may have the result, that the slurry-like composition cannot sufficiently be stabilized. In this particular case, adding small amounts of organic viscosity enhancers or rheological modifiers (e.g. polysaccharides in an amount of up to 0.1 wt.-% with respect to the solid components being present in the composition) can contribute to the delaying the sedimentation or agglomeration process until the gelation reaction takes place. Depending on the chemical composition, the shade or colour of the glass and/or glass ceramic material can be adjusted. This might facilitate the manufacturing of aesthetic dental restorations.

Glass or glass ceramic materials which may, for example, be used are generally available under the designations: "VM 9" from Vita Zahnfabrik, Bad Säckingen, Germany, "Cerabien Zr" from Noritake Inc., Japan, "Vintage" from Shofu, Japan; "ZIROX" from Wieland GmbH & Co.KG, Pforzheim, Germany and LM-ZrO2 from Chemichl, Liechtenstein.

The crystalline phase of the glass ceramic material may comprise leucit, lithium disilicate, zirconia and/or mica crystallites. Suitable lithium silicate glass ceramic materials are described e.g. in US 7,452,836 B2, US 6,306,784 B1, US 6,121,175. A typical composition comprises: SiO₂ in an amount of 55 to 71 wt.-%, Al₂O₃ in an amount of 5 to 16 wt.-%, B₂O₃, in an amount of 0.2 to 10 wt.-%, K₂O in an amount of 4 to 10 wt.-%, Na₂O in an amount of 3 to 14 wt.-% and optionally further components selected from CaO, F, P₂O₃, Li₂O, BaO and ZnO.

The glass and/or glass ceramic material does typically not comprise so-called reactive glass(es), that is, glass(es) which can undergo a neutralization or ion-exchange reaction with acidic substances. Reactive glasses are typically used in glass ionomer cement (GIZ) compositions.

A cement reaction is defined as the reaction of a reactive glass with a polyacid, which results in a hardened product typically within utmost 20 min upon mixing.

Preferred glasses and/or glass ceramic materials to be used according to the present invention comply with the requirements according to EN ISO 6872:2008. In contrast to reactive glasses used in glass ionomer cements, the glasses and/or glass ceramic materials to be used according to the present invention have a reduced solubility in diluted acetic acid, e.g. less than 100 µg/cm² (measured according to EN ISO 6872:2008, Table 1, Chapter 7.6).

The glass or glass ceramic powder can be present in an amount of at least 40 wt.-% or at least 60 wt.-% or at least 65 wt.-% with respect to the whole composition or mixture, respectively.

The glass or glass ceramic powder can be present in an amount up to 75 wt.-% or up to 80 wt.-% or up to 85 wt.-% with respect to the whole composition or mixture, respectively.

The glass or glass ceramic powder can be present in an amount ranging from 40 wt.-% to 85 wt.-%, but preferred from 60 wt.-% to 80 wt.-% and most preferred from 65 wt.-% to 75 wt.-%, with respect to the whole composition or mixture, respectively.

The nature and structure of the crosslinkable binder precursor is not particularly limited, either, unless the intended purpose cannot be achieved.

The crosslinkable binder precursor is typically able to form an inorganic network upon initiating a curing or hardening reaction. Inorganic network means that the network formed primarily contains Si-O bonds, but essentially no C-C bonds.

The curing or hardening reaction can be initiated e.g. by adjusting the pH value, either by adding acidic or basic reagents including those described in more detail below.

Preferably, the network of the binder has a similar or essentially identical chemical nature or composition as the chemical nature or composition of the glass/glass ceramic powder/particles used.

The crosslinkable binder precursor is preferably able to undergo or harden via a sol/gel reaction. That is, the binder precursor can undergo a hydrolysis and/or polycondensation reaction, thereby forming a colloid. A colloid can be described as a system comprising particles (e.g. having a size ranging from 1 nm to 1 µm) dispersed in a solvent.

Typically the crosslinkable binder precursor is a liquid at ambient conditions (23°C; 1013 mbar) or applied as an aqueous solution and can be characterized by at least one of the following features:
- density: 0.7 to 1.5 g/cm³ or 0.9 to 1.4 g/cm³,
- molecular mass: at least 100 g/mol or from 100 to 500 g/mol or from 150 to 250 g/mol (for molecular precursors),
- containing Si and O, and/or
- producing low boiling by- or condensation products during hardening, if any (e.g. boiling point below about 120 °C).

Specific examples of crosslinkable binder precursors which can be used include tetra alkyl (e.g. C1 to C4) orthosilicates (including tetramethyl orthosilicate (TMOS), tetraethyl orthosilicate (TEOS)), water glass and silica sol. That is, the binder precursor is preferably a material comprising Si-O moieties, but no Si-C moieties. Hydrolysable boron esters (e.g. boron triethoxide or tripopoxide) or boron salts convertible to the oxide by thermal or oxidative decomposition are typically not contained in an amount above 1, 2 or 5 wt.-%.

These crosslinkable binder precursors typically undergo a condensation reaction when the pH value is changed. E.g., orthosilicates typically start condensing at a pH value being in the basic range (e.g. from 8 to 14, or from 10 to 12), whereas water glass and silica sol start condensing at a pH value being in the acidic range (e.g. from 0 to 6 or from 2 to 4).

If desired, the progress of the condensation reaction of the crosslinkable binder system can be monitored using NMR techniques (e.g. ²⁹Si NMR).

The crosslinkable binder precursor is typically added in an amount ranging from 0.1 wt.-% to 10 wt.-%, but preferred from 0.5 wt.-% to 8 wt.-% and most preferred from 1.0 wt.-% to 6 wt.-%, with respect to the solids content of the mixture, respectively.

The crosslinkable binder precursor is typically added in an amount of at least 0.1 wt.-% or at least 0.5 wt.-% or at least 1.0 wt.-% with respect to the solids content of the mixture, respectively.

The crosslinkable binder precursor is typically added in an amount up to 10 wt.-% or up to 8 wt.-% or up to 6 wt.-% with respect to the solids content of the mixture, respectively.

Once added to the composition, the crosslinkable binder precursor typically starts forming a crosslinked network. The network forming rate typically depends on the amount of crosslinkable binder precursor used and the network-forming reaction conditions like the pH value. It can be sufficient if the pH value is slightly different from pH 7. The network-forming reaction can be accelerated if either acidic (H₃O⁺ producing substances) or basic (OH⁻producing substances) are added.

The appropriate amount of the binder precursor typically depends on the mass content of the slurry and the diameter of the glass and/or glass ceramic particles.

The composition may also comprise a rheological modifier. Thus, the presence of a rheological modifier is optional but can be preferred in some embodiments.

A rheological additive may facilitate the stabilisation of the composition, especially if the mean particle size of the glass and/or glass ceramic material is above 1 to 5 µm.

The rheological additive may also help to suspend solid particles (e.g. glass and/or glass ceramic material) in the liquid composition.

According to one embodiment of the invention, if present, the rheological additive is able to decrease the viscosity of the composition at constant shear rate. This means that a product subjected to shear will thin out, but once the shear forces are removed it will thicken back up.

It was found that this behavior can especially be advantageous for attaching small articles like dental facings and dental support structures to each other using a mating composition.

The nature and structure of the rheological modifiers is not particularly limited unless it is detrimental to the desired performance of the composition.

If present, the molecular weight of the rheological modifier should be high enough to provide for a sufficient sticky and viscous composition.

Typically, the molecular weight of the rheological modifier can be in a range from 500,000 to 100,000,000 or in a range from 600,000 to 70,000,000 or in a range from 800,000 to 60,000,000.

Specific examples of rheological modifiers which can be used include xanthan gum, polyethylene oxide (PEO) and mixtures thereof. Specific examples for polyethylene oxide include PEO 1,000,000 and PEO 8,000,000.

Xanthan gum is a polysaccharide. It can be produced by a process involving fermentation of glucose or sucrose using the bacterium *Xanthomonas campestris.* The backbone of the polysaccharide chain contains β-D-glucose units linked through the 1 and 4 positions. The side chain contains mannose and glucuronic acid. The overall chain consists of repeating modules of five sugar units. The molecular weight of xanthan typically varies within a range from 1 million up to 50 million depending upon how it is prepared.

It has been found that by adding xanthan gum to a liquid, typically an increase in its viscosity can be observed, even if only added in small quantities, e.g. on the order of 1 wt.-%.

If present, the rheological modifier is typically present in an amount of at least 0.01 wt.-% or at least 0.05 wt.-% or at least 0.1 wt.-% with respect to the whole composition.

The rheological modifier can be present in an amount up to 4 wt.-% or up to 2 wt.-% or up to 1 wt.-% with respect to the whole composition.

Typical wt.-% ranges for the rheological modifier include from 0.01 wt.-% to 4 wt.-% or from 0.05 wt.-% to 2 wt.-%.

The composition can contain further additives.

Additives which can be added include polyethylene glycol(s) (including e.g. those with a molecular weight below 10,000), thickening agent(s) (including e.g. selected from starch, methyl cellulose, ethyl cellulose, hydroxyl propyl cellulose, carboxy methyl cellulose), preserving agent(s) (including e.g. sorbic acid and benzoic acid), pigments or colorants and combinations and mixtures thereof.

According to a further embodiment, the inventive composition may also comprise a thickening agent being different from the rheological modifier.

The nature and structure of the thickening agent is not particularly limited unless it is detrimental to the desired performance of the composition.

The addition of a thickening agent can be beneficial in that a fine tuning of the layering properties of the composition can be achieved. E.g., the addition of methyl cellulose typically yields a stickier, more "honey like" consistency compared to an only e.g. xanthan gum containing composition. This may be beneficial for adaptations the tooth neck of a restoration if a layering with the slurry like composition might be necessary.

There is no need for a thickening agent to be present, if, however, a thickening agent is present, it is typically present in an amount of at least 0.01 wt.-% or at least about 0.05 wt.-% or at least 0.1 wt.-% with respect to the whole composition.

The thickening agent can be present in an amount up to 4 wt.-% or up to 2 wt.-% or up to 1 wt.-% with respect to the whole composition.

Typical wt.-% ranges for the thickening agent are from 0.01 wt.-% to 4 wt.-% or from 0.05 wt.-% to 2 wt.-%.

Thickening agents which can be used or added include starch, methyl cellulose (CAS number: 9004-64-2), ethyl cellulose (CAS number: 9004-57-3), hydroxyl propyl cellulose, carboxy methyl cellulose (CAS number 9000-11-7) and mixtures thereof.

Methyl cellulose is a methyl ether of cellulose, arising from substituting the hydrogen atoms of some of cellulose's hydroxyl groups -OH with methyl groups -CH₃, forming - OCH₃ groups.

Hydroxy propyl cellulose is an ether of cellulose in which some of the hydroxyl groups in the repeating glucose units have been hydroxypropylated forming -OCH₂CH(OH)CH₃ groups.

The inventive composition may also comprise further additives like a polyethylen glycol and/or glycerol and glycerol derivatives, being different (especially with respect to its chemical nature or formula) from the thickening agent(s) mentioned above. These components may facilitate the formulation of a storage stable composition.

Thus, the invention also relates to a composition comprising water, glass and/or glass ceramic material, a crossslinkable binder precursor, a rheological modifier and a polyethylene glycol.

Polyethylene glycol(s) which can be used typically have a molecular weight below 10,000 or below 8,000 or below 6,000.

The molecular weight of the polyethylene glycol can be in a range from 1,000 to below 10,000 or in a range from 2,000 to 8,000 or in a range from 3,000 to 6,000.

Polyethylene glycol (PEG; CAS Number: 25322-68-3) refers to an oligomer or polymer of ethylene oxide, having following structure: HO-(CH₂-CH₂-O-)ₙ-H, with n being typically in a range from 100 to 300.

The numbers that are often included in the names of PEGs indicate their average molecular weights, e.g. a PEG with n=9 would have an average molecular weight of approximately 400 and would be labelled PEG 400.

Most PEGs include molecules with a distribution of molecular weights, i.e. they are polydisperse. The size distribution can be characterized statistically by its weight average molecular weight (Mw) and its number average molecular weight (Mn), the ratio of which is called the polydispersity index (Mw/Mn). Mw and Mn can be measured by mass spectroscopy

The two names polyethylene glycol (PEG) and polyethylene oxide are chemically synonymous, but historically PEG is referred to shorter polymers with a molecular weight < 20,000, and polyethylene oxide to high-molecular adducts.

The polyethylene glycol can be present in an amount of at least 0.2 wt.-% or at least 0.4 wt.-% or at least 0.5 wt.-% with respect to the whole composition.

Polyethylene glycol can be present in an amount up to 10 wt.-% or up to 5 wt.-% or up to 1 wt.-% with respect to the whole composition.

Typical wt.-% ranges for the polyethylene glycol, if present, include from 0.1 wt.-% to 10 wt.-% or from 0.4 wt.-% to 5 wt.-%.

Specific examples of polyethylene glycol which can be used include PEG 200, PEG 285-315, PEG 380-420, PEG 570-630, PEG 950-1050, PEG 1305-1595, PEG 1900-2200, PEG 3015-3685.

The composition may also comprise pigments. Pigments which can be used include the oxides of Cr, Mn, Fe, V, Co, Sb, Ni, Sn, Zn, combination and mixtures thereof. The use or addition of pigments to the composition can be beneficial in that, in particular after a heating or sintering step tooth coloured product(s) can be provided, wherein the term "tooth coloured" refers to the Vita colour scheme known to the person skilled in the art.

The composition may also comprise a preserving agent including e.g. sorbic acid or benzoic acid. The addition of a preserving agent may further improve the storage stability.

According to one embodiment, the composition may comprise the components in the following amounts:
a. liquid(s): from 15 wt.-% to 60 wt.-%, or from 20 wt.-% to 40 wt.-%,
b. glass and/or glass ceramic material(s): from 40 wt.-% to 85 wt.-%, or from 60 wt.-% to 80 wt.-%,
c. crosslinkable binder precursor(s): from 0.1 wt.-% to 10 wt.-%, or from 0.5 wt.-% to 8 wt.-%, or from 1 to 6 wt.-%,
d. rheological modifier(s) (with a molecular weight above 500,000): from 0.01 wt.-% to 4 wt.-%, or from 0.05 wt.-% to 2 wt.-%,
e. polyethylene glycol(s) (with a molecular weight below 10,000): from 0 wt.-% to 10 wt.-%, or from 0.1 wt.-% to 5 wt.-%, and
f. thickening agent(s): from 0.01 wt.-% to 4 wt.-%, or from 0.05 wt.-% to 2 wt.-%,
g. pigment(s) or colorant(s): from 0.01 wt.-% to 2 wt.-%, or from 0.05 wt.-% to 1 wt.-%,
h. preserving agent(s): from 0.01 wt.-% to 2 wt.-%, or from 0.05 wt.-% to 1 wt.-%.

The amount of crosslinkable binder precursor used allows adjusting or influencing the gel time and the toughness of the dried material. The amount of powder and water used allows adjusting the density of the dried blocks.

According to a specific embodiment of the invention, the composition comprises or essentially consists of water, glass and/or glass ceramic material and a crosslinkable binder precursor.

According to another specific embodiment of the invention, the composition comprises water, glass and/or glass ceramic material, crosslinkable binder precursor and a rheological modifier (e.g. polyethylene glycol (Mw < 1000; e.g. Mw = about 400), xanthan gum and, if desired a thickening agent like e.g. methyl cellulose and further optionally a preserving agent (e.g. sorbic acid).

Especially for fusing a dental core to a dental facing, the combination of xanthan gum with a thickening agent like methyl cellulose can be advantageous. This combination may provide for a good balance between sufficient viscosity, sticking behaviour and the ability to create thin layers.

The ratio (with respect to weight) of liquid to crosslinkable binder precursor is typically in a range of 20:1 to 1:1 or 10:1 to 3:1. If the ratio is outside this range, it might happen that the obtained result does not show the desired properties.

The ratio (with respect to weight) of crosslinkable binder precursor to glass or glass ceramic powder can be in a range of 1:200 to 1:5 or 1:100 to 1:10. If the ratio is outside this range, it might happen that the obtained result does not show the desired properties.

The composition can be produced by mixing the individual components either by hand or using a mechanical mixing device. Speed mixers were found to be beneficial.

The pH value of the composition has typically to be adjusted to a suitable value in order to start or initiate the gelation process of the precursor used.

If metal alkoxides are used as gel precursors or part thereof, the composition may already have an appropriate pH value to promote hydrolysis of the precursor. It has been found that using deionized water as liquid or dispergent can be sufficient, since the dispersed glass or glass ceramic particles will by itself provide for a pH value in the required pH region (e.g. pH value above 7).

According to one embodiment, the composition can be obtained by a process comprising the steps of:
i) providing a liquid,
ii) adding the glass or glass ceramic powder
iii) if needed or desired, adjusting the liquid to a pH value suitable to start the condensation reaction, depending on the crosslinkable binder precursor used (e.g. 10 to 12 for TMOS or 2 to 4 for water glass), and
iv) adding the crosslinkable binder precursor,
wherein steps ii) and iii) can also be carried out in reverse order.

The process above can be advantageous to provide compositions, which essentially do not contain any organic substance(s). In this particular embodiment, all components used are miscible with water.

If needed or desired, the pH value can be adjusted by using conventional basic reagents like NaOH, KOH or NH₃ containing solutions or acidic reagents like acetic acid, HCl, oxalic acid or HNO₃ containing solutions, wherein the pH value is typically determined during the adjustment step. The pH value can be determined by e.g. pH sensitive paper or electronic equipment (e.g. pH electrode). If strong acids or bases are employed, determination of the pH value can also be obtained via calculation from the amount of acid used.

The crosslinkable binder precursor is typically added rapidly while the composition is stirred. The addition of the crosslinkable binder precursor usually marks the starting point of the sol-gel reaction caused by the reaction of the precursor molecules. During the sol-gel reaction typically an inorganic network is formed.

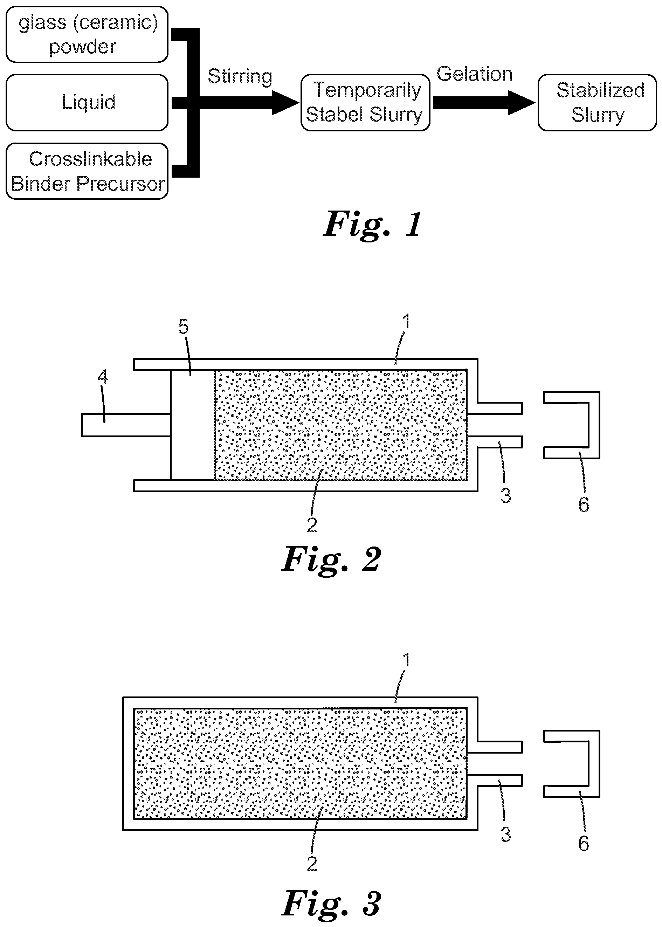

Figure 1 outlines a possible production step. Glass (ceramic) powder, solvent and crosslinkable binder precursor are combined and stirred thereby obtaining a temporarily stable slurry/composition. The crosslinkable binder precursor starts the gelation process having the result that a permanently stabilized slurry is obtained, wherein the glass (ceramic) powder do not show sedimentation any more.

The invention features a process for producing a dental restoration or part thereof, wherein the process comprises the steps of providing the container comprising the composition as described in the present text and applying the composition to the surface of a dental article.

If desired, the composition can be repeatedly applied to the surface of a dental article, in order to obtain a layered structure. If different compositions, especially compositions with different colours or pigments, are applied on the surface of a dental article the shape and looking of the dental article can be adjusted to the looking of a natural tooth or tooth structure.

It has been found that due to the rheological behaviour and storage stability, the inventive composition can also advantageously be used for veneering dental support structures. The composition is sufficiently viscous and does typically show no dropping. Moreover, the composition can be used as it is. Usually, there is no need for an additional mixing step.

The application of the composition to the surface of the dental article can be done e.g. by dipping, brushing or painting (e.g. using a brush, spatula or similar equipment). However, other suitable means can be used, as well.

It has also been found that the composition is particularly useful for modelling, forming or adapting dental restorations, including forming, adapting or modelling shoulders, pontics (i.e. a structure bridging a gap between two restorations, thereby forming a dental bridge) and/or cusps.

Thus, described is also a process of forming shoulders, pontics and/or cusps on dental restorations, especially on dental support structures (including dental support structure comprising either metal or ceramic materials), thereby using the container comprising the composition as described in the text of the invention.

As generally known, these steps are typically done by a dental technician in a dental lab.

The invention is also directed to a process for producing a dental restoration comprising the following steps:
- providing a dental facing and a dental support structure, both having an upper and a lower surface,
- optionally applying a silica layer on either or both of the surfaces of the dental facing or dental support structure,
- optionally wetting the surface of the dental facing with a liquid comprising or consisting essentially of or consisting of water,
- attaching the dental facing with its lower surface to the upper surface of the dental support structure, comprising the step of placing the composition contained in a container as described in the present text between the lower surface of the dental facing and the upper surface of the dental support structure, thereby obtaining a dental restoration intermediate.

If desired, the dental restoration intermediate can be sintered afterwards.

The application of the composition on the surface of the dental articles to be mated can be done with an application instrument such as a spatula or a brush. Alternatively, the composition can be applied directly out of the nozzle of the sealable container.

The term "dental facing" includes sintered dental facings and dental facing precursors (not sintered to final density yet). A dental facing typically has an upper and a lower surface, wherein the lower surface typically has a concave shape.

The dental support structure has an upper and lower surface, too. Ideally, the shape provided by the lower surface of the dental facing corresponds essentially to or is proportionally enlarged to the shape provided by the upper surface of the dental support structure. The shape of the upper surface of the dental support structure is typically convex.

It can be advantageous, if the parts to be mated or attached are treated with a water-containing liquid first, before the mating composition is applied. This can be done either by applying (e.g. by spraying or brushing) water on the respective surfaces and/or by placing the article in a water containing device. Doing this may enhance the wetting ability of the surfaces of the articles to be mated with respect to the inventive mating composition. Another benefit can be seen in the fact that a smooth application of the inventive mating composition can be accomplished. A wetted porous surface is typically more resistant to absorbing further liquid from another composition (e.g. inventive mating composition). If the surface of the dental facing is not treated with a wetting agent before the application of the inventive mating composition, it has sometimes been observed that the liquid components of the mating composition are soaked by the dental facing and thus may hamper the flowing of the inventive mating composition. This may result in a defective or less perfect dental restoration after a firing or heating step.

Ideally the concave mould provided by the lower surface of the dental facing should be filled to at least 30 vol.-% or at least 50 vol.-%.

The inventive composition can be applied on the lower surface of the dental facing only or on the upper surface of the dental frame only or on both, the lower surface of the dental facing and on the upper surface of the dental frame.

The surfaces of the parts to be mated or attached are typically pressed slightly (finger pressure) against each other.

To remove excess mating composition, if present, the dental restoration intermediate can be placed on a soaking surface (e.g. paper tissue) and is typically left there for a couple of minutes.

A dental facing precursor for example can be characterized by one or more of the following features:
a. comprising an open-celled material,
b. comprising a glass and/or glass ceramic material,
c. being proportionally enlarged relative to a sintered facing by a magnification factor of between 1.12 to 1.9
d. coefficient of thermal expansion: from 8 ^{∗} 10⁻⁶K⁻¹ to 15.8 ^{∗} 10⁻⁶K⁻¹,
e. the material density being in a range of from 0.6 g/cm³ to 2.5 g/cm³.

The chemical composition of the material the dental facing is made of or comprised of often corresponds or is nearly identical to the chemical composition, the glass and/or glass ceramic material described in the text above is comprised of.

According to another embodiment, the glass and/or glass ceramic material used for producing the dental facing can be characterized by at least one of the following features:
- Density: 2.0 to 2.8 g/cm³ or 2.2 to 2.6 g/cm³ and/or
- Glass transition temperature: 500 to 600 °C or 520 to 580 °C, preferably about 550 °C.

Glass or glass ceramic materials which may, for example, be used for manufacturing a blank and/or a dental facing precursor are generally available under the designations:
- "VMK 95" and "Omega 900" from Vita Zahnfabrik, Bad Säckingen, Germany;
- "IPS Classic" and "IPS d.Sign" from Ivoclar Vivadent, Liechtenstein;
- "Vintage" from Shofu, Japan; and
- "REFLEX" from Wieland GmbH &Co.KG, Pforzheim, Germany.
- "VM9" from Vita Zahnfabrik, Bad Säckingen, Germany
- "LAVA Ceram" from 3M ESPE, Seefeld, Germany
- "Cerabien Zr" from Noritake, Japan
- "LM ZrO2" from Chemichl AG, Liechtenstein.

The dental facing as it is referred to in this specification may be substantially free of cells, however may comprise up to 16 cells per mm². Preferably, the dental facing may comprise 4 to 8 cells per mm². The cells preferably have a diameter of less than 150 µm, and more preferably a diameter of less than 100 µm and most preferably a diameter of less than 40 µm. In a particular embodiment the facing has less than 16 cells per mm² with a diameter of less than 150 µm, wherein not more than 6 cells have a diameter of between 40 and 150 µm. The unit "cells per mm²" is related to the number of cells present on a cross section according to the test method as defined in DIN 13925.

The dental facing precursor preferably comprises an open-celled material. The term "open-celled" within this context typically relates to an "open porosity" according to the mercury porosimetry as defined in DIN 66133 of between 6% and 35%, in particular of between 15% and 35%, and in more particular of between 30% and 35%.

The raw breaking resistance of the pre-sintered material or the dental facing precursor as referred to in this specification is preferably in a range of 3 to 15 MPa, more preferably in a range of 4 to 12 MPa, and preferably 5 MPa to 9 MPa according to the "punch on three ball test" as specified in ISO 6872.

The bending strength of the sintered material or the dental facing as referred to in this specification is preferably in a range of 50 to 400 MPa, in more particular in a range of 50 to 120 MPa according to the "punch on three ball test" as specified in ISO 6872.

The material the dental facing is made of may be selected to provide a certain translucency. Typically the translucency is specified by the opacity of a material relative to daylight. Typical ranges of the opacity of the sintered material or the facing are 50% to 60% (typically corresponding to natural dental enamel), 60% to 80% (typically corresponding to natural dentine) and 80% to 90% (typically corresponding to natural opaque dentine).

The dental facing precursor is placed with its inner surface (typically concave shape) on the outer surface of the dental support structure (typically convex shape). The inner surface of the facing precursor may be a proportionally or similarly dimensioned counterpart to the outer surface of the dental support structure, and more specifically the inner surface of the facing precursor may be a proportionally enlarged counterpart of the outer surface of the dental support structure, to permit the former to fit over the latter with any appropriate clearance.

It can be advantageous, to pre-treat the surface either of the dental facing or of the dental support structure before the inventive mating composition is applied, wherein pre-treating the surface of the dental support structure is preferred, especially if the dental support structure comprises or essentially consists of a ceramic (e.g. zirconia or alumina) material. A suitable pre-treatment step includes the formation of a silica layer on either or both of these surfaces.

This can be done, e.g. by sand-blasting or rubbing the surface with a composition comprising
a) 0.01 to 90 wt.-% of a material (optionally silanized) with a particle size < about 5 mm and a hardness exceeding that of the substrate surface;
b) 20 to 100 wt.-% of a silanized silicon-containing material having a particle size of 2 to 200 mm;
c) the remainder of a sand blasting composition having a particle size of < 5 mm.

Such a process is described e.g. in US 5,024,711. The respective equipment can be obtained from 3M ESPE under the brand Rocatec™.

Pre-treating the surface e.g. by applying a silica layer may improve the wetting characteristics of the surface and facilitate the formation of thin layers.

The dental facing precursor and the dental support structure are fitted with the inventive mating composition arranged between mated surfaces of the dental support structure and the dental facing precursor.

Besides as function to mate the individual parts, the inventive mating composition may further provide for compensating tolerances within a gap between the individual parts. In case a dental restoration precursor formed by use of a mating composition is sintered, the mating composition typically dries during sintering and the remaining particles fuse and form an intermediate layer between the dental facing and the dental support structure.

It can be beneficial, if the value of the coefficient of thermal expansion of the dental facing or the material the dental facing is made of is smaller than the thermal expansion of the material the dental support structure is made of. This may help to increase the compressive strength of the facing and might facilitate the provision of a durable dental restoration.

A typical dental support structure can be characterized by one or more of the following features:
a. comprising a ceramic material or metal,
b. coefficient of thermal expansion: from 8 ^{∗} 10⁻⁶K⁻¹ to 20 ^{∗} 10⁻⁶K⁻¹,
c. the material density of the dental support structure being in a range of from 5 g/cm³ to 19 g/cm³ or from 5 g/cm³ to 10 g/cm³ or from 5 g/cm³ to 7 g/cm³.

Typical ceramic materials which can be used include zirconia, alumina and combinations thereof.

With respect to zirconia, yttrium doped tetragonal stabilized zirconia is preferred. This material is often also referred to as YTZP and commercially available form e.g. Tosoh Comp., Japan.

Metal for manufacturing a dental support structure can be characterized by at least one of the following features:
- Coefficient of thermal expansion of the metal the metallic frame is made of typically ranges from 9.6 ^{∗} 10⁻⁶ K⁻¹ to 17.3 ^{∗} 10⁻⁶ K⁻¹. Other suitable ranges include from 9.6 ^{∗} 10⁻⁶ K⁻¹ to 15.2 ^{∗} 10⁻⁶K⁻¹ and 13.8 ^{∗} 10⁻⁶ K⁻¹ to 15.2 ^{∗} 10⁻⁶ K⁻¹ and 16 ^{∗} 10⁻⁶ K⁻¹ to 17.3 ^{∗} 10⁻⁶ K⁻¹.
- Being selected from the group consisting of Ti, Au, Pt, Pd, Ag, Zn, Co, Cr, Mo, W, Ni and combinations and alloys thereof.

Suitable dental alloys can be obtained from Argen Comp., Wieland Comp., Bego Comp., Dentaurum Comp. and DeguDent Comp.

The process described above can also comprise a heating step, wherein the heating step can be characterized by one or more of the following parameters:
a. Temperature: between 500°C to 1200°C or between 600°C to 1050°C,
b. Duration: between 1 min to 1 h or from 10 min to 30 min.
c. Pressure: between 25 and 1025 mbar.

Thus, the dental restoration intermediate may be heated to a temperature of 500 °C to 1000 °C for a time period of 30 s to 10 min. The heating step may further be done at a pressure of between 25 and 1025 mbar.

The heating step can be repeated if desired.

Heating the dental facing precursor to a sufficient high temperature typically results in densification of the porous material to a less porous material (or a material having less cells) having a higher density. In some cases the heating may also cause changes of the material phase composition (for example, a partial conversion of an amorphous phase toward a crystalline phase).

During the heating step, the liquid components of the mating composition evaporate or are combusted and the remaining glass and/or glass ceramic material, if present, remains. Depending on the temperature chosen, the glass and/or glass ceramic material may fuse with material the dental facing is made of.

It the dental facing contains a porous and open-celled structure, the gases created during the heating step may more easily migrate through the pores of the dental facing precursor and facilitate the provision of a homogeneous, essentially defect free interface layer between the dental support structure and the dental facing.

If desired, the combustion behaviour of the inventive composition can be examined as follows:
A liquid/powder mixture (containing a glass and/or glass ceramic material) is used to join a milled and sintered zirconia containing dental support structure (e.g. LAVA™; 3M ESPE Comp.) to a dental facing precursor material. The dental facing precursor material is wetted with deionized water. The liquid/powder mixture is applied with a brush to the surface of the dental facing precursor. Dental support structure and dental facing precursor are put together and left to dry. After drying, the dental restoration intermediate is sintered to full density at about 780 °C in an Austromat™ 3001 (from Dekema). Discoloration (e.g. greyish appearance) of the fired restoration showed incomplete combustion of the organic components of the slurry, while no discoloration proves combustion.

The invention is also directed to a dental restoration obtainable or obtained by the process described above. Such a dental restoration typically shows less defects (e.g. voids, air bubbles, etc.) after a heating step in the region between the dental facing precursor and the dental facing precursor, that is the region where the inventive composition has been applied.

A typical procedure for producing a dental restoration is described herein below:
A mixture is prepared by mixing a glass and/or glass ceramic material with a liquid comprising a rheological additive as described above, optionally polyethylene glycol and de-ionized water. The powder to liquid ratio is 2.5 : 1 by weight. Then the dental facing having a concave inner surface is filled to 2/3 with the mixture and put on top of the upper surface of the dental support structure, thereby obtaining a dental restoration intermediate.

The dental restoration intermediate is laid on a water absorbing paper. After about 2-3 min drying time, an excess of composition, if any, is removed and missing composition added, if desired. After drying, the dental restoration intermediate is fired in an oven to obtain a dental restoration. The outer surface of the obtained dental restoration can be glazed using a glazing composition, if desired.

Described is also a kit of parts comprising in separate parts
a. a container containing the composition as described in the text of the invention and
b. an application device.

Suitable application devices include brushes, spatulas and all means which allow for an application of the composition to small objects especially those having concave and/or convex surfaces.

The container may have the shape of a bottle, (screw) tube, flasks, syringe, capsule, cartridge and the like. It can also be preferred, if the composition is stored in a receptacle (e.g. a blister package) which also allows for using the composition as it is, that is, without the need for an additional mixing step.

The container can be a disposable container ("single use container").

The container is preferably sealed during storage and before use and re-sealable after use.

Sealing of the container can be accomplished e.g. by using removable caps, stoppers or foils, including self-adhesive foils.

The volume of the container is not particularly limited. Typically, the container has a volume in the range of 0.1 to 11 or from 0.5 ml to 100 ml or from 1 ml to 10 ml.

The container typically contains a delivery system like a nozzle.

The invention also relates to a kit of parts comprising at least two containers as described in the present text, wherein at least one of the compositions contained in the container comprises in addition a colorant or pigment.

Thus, the kit may comprise a variety of different compositions e.g. in different volumes and/or shades. For example, the kit may comprise 1 container comprising an uncoloured or un-pigmented composition and from 1 to 20 containers comprising coloured or pigmented compositions (e.g. compositions adapted to different tooth colours). The kit may also comprise at least 2 containers comprising different coloured or pigmented compositions.

Suitable embodiments for containers which can be used are shown in Figures 2 and 3.

Fig. 2 shows a syringe like container (A) with a receptacle (1) having preferably a rigid hull. In the receptacle the composition (2) is stored until use. The container contains a nozzle (3) and a plunger (4) with sealing lips (5). The container (1) is sealed with a removable cap (6).

Fig. 3 shows an alternative container, however, without a plunger. In this embodiment the receptacle is preferably made of a flexible material to facilitate delivery of the composition (2) through the nozzle (3) by using pressure on the outer walls of the receptacle (1).

The composition does typically not comprise one or more of the following components selected from the group consisting of: ethylenically unsaturated compounds with or without acid functionality, cross-linkable polyacid(s), initiator(s), fluoroaluminiasilicate glass(es) and mixtures thereof.

Cross-linkable polyacids are e.g. polyacrylic acid, polygalacturonic acid, polyethylene-co-maleic acid, that is, acids which can be obtained by polymerizing polymerizable acidic monomers. Thus, polyacids bear a huge number of acidic groups, typically at least one acidic group per polymerized acidic monomer.

The composition does typically also not contain reactive organic monomers (e.g. monomers containing (meth)acrylates or epoxy groups).

More particularly, the composition does typically not comprise one or more of the following components selected from: ethylenically unsaturated compounds with or without acid functionality, cross-linkable polyacid(s), initiator(s), fluoroalumina silicate glass(es) and mixtures thereof.

Cross-linkable polyacids are e.g. polyacrylic acid, polygalacturonic acid, polyethylene-co-maleic acid, that is, acids which can be obtained by polymerizing polymerizable acidic monomers. Usually, polyacids bear a huge number of acidic groups, typically at least one acidic group per polymerized acidic monomer.

Thus, the composition can neither be regarded as a radical curable composition nor as a glass ionomer cement like composition.

Moreover, the addition or presence of an initiator (e.g. photo or redox initiator) for starting the hardening process of the inorganic binder precursor is typically not needed. The hardening process can be initiated by adjusting the pH value or simply by employing a diluted acidic/basic solution.

The composition does typically also not contain components producing a toxic, injurious, or immunological response in living tissue or components or additives which jeopardize the intended purpose to be achieved with the present invention.

Thus, for example components or additives added in an amount which finally results in a non-tooth-coloured article are usually not contained in the final dental restoration. Typically, an article is characterized as tooth coloured if it can be allocated a colour from the Vita™ colour code system, known to the person skilled in the art.

### Examples

The following examples are given to illustrate this invention.

Unless otherwise indicated, all parts and percentages are on a weight basis, all water is deionized water, and all molecular weights are weight average molecular weight. Moreover, unless otherwise indicated all experiments were conducted at ambient conditions (23°C; 1013 mbar).

### Measurements

### Density

If desired, density measurements can be performed by determination of the volume of symmetric blocks and their mass.

### Particle Size

If desired, the mean particle size can be determined using a commercially available granulometer (Laser Diffraction Particle Size Analysis Instrument, CILAS 1064; CILAS Comp.) according to the instruction of use provided by the manufacturer.

### pH Value

If desired, the pH-value can be determined using a mobile pH-indicator paper available from Merck KGaA, Darmstadt Germany (pH 0-14, pH indicator strips, non bleeding, Art. No. 1.09535.0001). Additionally, pH measurements can be performed by employing a pH electrode and measurement device "744 pH meter" from Metrohm.

### Viscosity:

If desired, the viscosity of a liquid/powder mixture prepared according to the examples below can be measured with a viscosimeter MCR301 (from Anton Paar Comp.). For the measurement, a portion of the liquid/powder mixture was placed between two steel discs with a diameter of 8 mm and a gap of 1 mm at a temperature of 23°C. The gap was filled completely with the liquid/powder mixture. Excess liquid/powder mixture was removed. The shear rate between the rotating discs d(gamma)/dt was set constantly to 50 s⁻¹. 100 measurements of the viscosity were performed, one every 4 seconds. To avoid an undesired drying of the slurry during the measurement a ribbon of wetted paper tissue is laid around the discs in order to raise the humidity.

A decrease of viscosity during a constant shear stress is preferred, because such behaviour is typically beneficial when joining a dental support structure to a dental facing to produce a dental restoration.

### Abbreviations

Table 1 describes some of the components used in the Examples below in more detail.

**Table 1**

| Name | Description |
|---|---|
| TMOS | Tetra methyl orthosilicate |
| TEOS | Tetra ethoxy orthosilicate |
| LM-ZrO₂ | 97-100 % SiO₂, Al₂O₃, K₂O, Na₂O, CaO, La₂O₃, ZnO₂, B₂O₃, CeO₂, SrO, SnO₂, Li₂O, F, P₂O₅, 0-3 % shading components |

### Example 1:

A solution was prepared by adding 0.012 g of xanthan gum (Jungbunzlauer) to 3.7 ml of deionized water. 10 g of glass powder (GM/LM-Zr Incisal 2, average particle diameter 19 µm, Chemichl, Liechtenstein) was dispersed into the solution and the slurry obtained was stirred at moderate speed. 0.4 ml of TMOS (tetramethyl orthosilicate) was added and the slurry was stirred more rapidly until the TMOS was distributed homogeneously in the reaction mass. Hereinafter, stirring was stopped and the composition was let alone for about 24 h during which gelation occurred. Finally, the composition was poured into a transparent tube and sealed and stored.

Result: No sedimentation of the glass powder was observed with the human eye over a period of 8 months.

### Example 2:

A solution was prepared of 0.01 g xanthan gum (from Jungbunzlauer) and 3.1 ml of deionized water. To this solution 10.0 g of glass powder (GM/LM-Zr Incisal 2 from Chemichl, Liechtenstein; average particle diameter: 19 µm) was added. The resulting slurry was stirred until the glass powder was distributed homogeneously. Then, 0.4 ml of TEOS (tetraethoxy silane, 98% from Fluka, Sigma-Aldrich, Germany) was added. The vessel with the slurry was covered to avoid evaporation of solvent. The mixture was stirred until the TEOS phase had disappeared. After that, the mixture was transferred to a sealable container and stored.

Result: No sedimentation of the glass powder was observed with the human eye over a period of 3 months.

### Example 3:

A solution was prepared of 1.1 ml acetic acid (32 %, from VWR, Germany) and 1.8 ml of deionized water. 10.0 g of glass powder (GM/LM-Zr Incisal 2 from Chemichl, Liechtenstein; average particle diameter: 19 µm) was added. The resulting slurry was stirred until the glass powder was distributed homogeneously. Then, 0.4 ml of water glass (sodium silicate solution, Sigma-Aldrich, Germany) was added. The vessel with the slurry was covered to avoid evaporation of solvent. The mixture was stirred until the water glass was distributed homogeneously. After that, the mixture was transferred to a sealable container and stored. Because the sol-gel reaction was proceeding so quickly, no rheological modifier had to be added.

Result: No sedimentation of the glass powder was observed with the human eye over a period of 1 month.

### Comparative Example

A comparison experiment had shown that using xanthan gum alone at the concentration given above (cf. Example 1, however, without a crosslinkable binder precursor) is not suitable to prevent sedimentation of the glass powder from the slurry for more than about 4-5 days.

## Claims

1. An ex vivo process for producing a dental restoration or part thereof comprising the steps
a. providing a container containing a composition to be applied on a dental article, the composition comprising glass and/or glass ceramic particles being contained in a gel, the gel being obtainable by a sol/gel reaction of a Si and O containing inorganic crosslinkable binder precursor being contained in a liquid, the container being sealable or sealed,
b. applying the composition contained in the container to the surface of a dental article,
wherein the dental article or part thereof is a dental support structure, a dental facing precursor, an abutment or a dental implant and
wherein the crosslinkable binder precursor of the composition is selected from orthosilicates, silica sol, water glass, and mixtures thereof.

2. The process according to claim 1, wherein the crosslinkable binder precursor of the composition is **characterized by** at least one of the following features:
∘ liquid at ambient conditions or applicable as aqueous solution or dispersion,
∘ density: from 0.7 to 1.5 g/cm³,
∘ molecular mass: at least 100 g/mol, and/or
∘ producing only low boiling condensation or by- products during a crosslinking reaction.

3. The process according to any of the preceding claims, wherein the composition is **characterized by** a pH-value (aqueous composition) being in the basic range if the crosslinkable binder precursor comprises an orthosilicate or being in the acidic range if the crosslinkable binder precursor comprises water glass, silica sol, or mixtures thereof.

4. The process according to any of the preceding claims, wherein the glass or glass ceramic particles in the composition are **characterized by** at least one of the following features:
∘ mean particle size: range from 5 µm to 60 µm, (measured with laser diffraction; 23°C),
∘ melting temperature: below 1000 °C,
∘ density: 2.0 to 2.8 g/cm³.

5. The process according to any of the preceding claims, wherein the composition comprises in addition at least one or more of the following components:
a. a rheological modifier with a molecular weight above 500,000,
b. polyethylene glycol with a molecular weight below 10,000,
c. thickening agent being different from the rheological modifiers selected from starch, methyl cellulose, ethyl cellulose, hydroxyl propyl cellulose, carboxy methyl cellulose, combinations and mixtures thereof,
d. pigments or colorants,
e. preserving agent,
and mixtures thereof.

6. The process according to any of the preceding claims, wherein the composition is **characterized by** one or more of the following features:
∘ viscosity of the composition without glass and/or glass ceramic material: being in the range of 10 to 1,000 mPas, measured at a temperature of 23°C with a shear rate s d(gamma)/dt of 50 s⁻¹,
∘ content of organic components: being below 7 wt.-%, with respect to the weight of the whole composition.

7. The process according to any of the preceding claims, wherein the composition comprises the components in the following amounts:
a. liquid: from 15 wt.-% to 60 wt.-%,
b. glass and/or glass ceramic material: from 40 wt.-% to 85 wt.-%,
c. crosslinkable binder precursor: from 0.1 wt.-% to 10 wt.-%,
d. rheological modifier: from 0 wt.-% to 4 wt.-%,
e. polyethylene glycol: from 0 wt.-% to 10 wt.-%,
f. thickening agent: from 0 wt.-% to 4 wt.-%,
g. pigments or colorants from 0 wt.-% to 2 wt.-%,
h. preserving agent: from 0 wt. -% to 2 wt.-%,
wt.-% with respect to the weight of the whole composition.

8. The process according to any of the preceding claims, wherein the dental facing precursor is **characterized by** one or more of the following features:
• the facing precursor comprising an open-celled material,
• the facing precursor comprising a glass and/or glass ceramic material,
• the facing precursor being proportionally enlarged relative to a sintered facing by a magnification factor of between 1.12 to 1.9,
• coefficient of thermal expansion: from 8 ^{∗} 10⁻⁶K⁻¹ to 15.8 ^{∗} 10⁻⁶K⁻¹,
• the material density of the facing precursor being in a range of from 0.6 g/cm³ to 2.5 g/cm³.

9. The process according to claim 8, wherein the dental support structure is **characterized by** one or more of the following features:
• the dental support structure comprising a ceramic material or metal,
• coefficient of thermal expansion: from 8 ^{∗} 10⁻⁶K⁻¹ to 15.8 ^{∗} 10⁻⁶K⁻¹,
• the material density of the dental support structure being in a range of from 5 g/cm³ to 19 g/cm³.

10. The process according to claim 8 comprising the following steps:
• providing a dental facing precursor and a dental support structure, both having an upper and a lower surface,
• attaching the dental facing precursor with its lower surface to the upper surface of the dental support structure, comprising the step of placing the composition as described in any of claims 1 to 8 between the lower surface of the dental facing precursor and the upper surface of the dental support structure
• and optionally a heating step, wherein the heating step is **characterized by** one or more of the following features:
- temperature: between 500 °C to 1200 °C,
- duration: between 1 min to 1 h,
- atmospheric pressure: between 25 and 1025 mbar.

11. Use of a composition being contained in the container as described in any of claims 1 to 7 for veneering, modelling, forming or adapting a dental article, the composition comprising glass and/or glass ceramic particles being contained in a gel, the gel being obtainable by a sol/gel reaction of a Si and O containing inorganic crosslinkable binder precursor being contained in a liquid, wherein the crosslinkable binder precursor of the composition is selected from orthosilicates, silica sol, water glass, mixtures thereof, and wherein the dental article is selected from a dental support structure, a dental facing precursor, an abutment or a dental implant.

12. Kit of parts comprising at least two containers as described in any of claims 1 to 7, wherein at least one of the compositions contained in the container comprises in addition a colorant or pigment, the composition comprising glass and/or glass ceramic particles being contained in a gel, the gel being obtainable by a sol/gel reaction of a Si and O containing inorganic crosslinkable binder precursor being contained in a liquid, wherein the crosslinkable binder precursor of the composition is selected from orthosilicates, silica sol, water glass, and mixtures thereof.

## Patentansprüche

1. Ein Ex-vivo-Verfahren zum Herstellen einer Zahnrestauration oder eines Teils davon, umfassend die Schritte
a. das Bereitstellen eines Behälters, der eine auf einen Dentalartikel aufzutragende Zusammensetzung enthält, wobei die Zusammensetzung Glas- und/oder Glaskeramikteilchen umfasst, die in einem Gel enthalten sind, wobei das Gel durch eine Sol/Gel-Reaktion eines Si und O erhältlich ist und anorganischen vernetzbaren Bindemittelvorläufer enthält, der in einer Flüssigkeit enthalten ist, wobei der Behälter versiegelbar oder versiegelt ist,
b. Auftragen der in dem Behälter enthaltenen Zusammensetzung auf die Oberfläche eines Dentalartikels,
wobei der Dentalartikel oder ein Teil davon eine Zahnträgerstruktur, ein Zahnverblendungsvorläufer, ein Abutment oder ein Dentalimplantat ist und
wobei der vernetzbare Bindemittelvorläufer der Zusammensetzung aus Orthosilikaten, Kieselsol, Wasserglas und Mischungen davon ausgewählt ist.

2. Das Verfahren nach Anspruch 1, wobei der vernetzbare Bindemittelvorläufer der Zusammensetzung durch mindestens eines der folgenden Merkmale **gekennzeichnet ist:**
∘ flüssig bei Umgebungsbedingungen oder auftragbar als wässrige Lösung oder Dispersion,
∘ Dichte: 0,7 bis 1,5 g/cm³,
∘ Molekularmasse: mindestens 100 g/mol, und/oder
∘ nur niedrigsiedende Kondensation oder Nebenprodukte während einer Vernetzungsreaktion produzierend.

3. Das Verfahren nach einem der vorstehenden Ansprüche, wobei die Zusammensetzung durch einen pH-Wert (wässrige Zusammensetzung) **gekennzeichnet ist,** der im basischen Bereich liegt, wenn der vernetzbare Bindemittelvorläufer ein Orthosilikat umfasst, oder im sauren Bereich liegt, wenn der vernetzbare Bindemittelvorläufer Wasserglas, Kieselsol oder Mischungen davon umfasst.

4. Das Verfahren nach einem der vorstehenden Ansprüche, wobei die Glas- oder Glaskeramikteilchen in der Zusammensetzung durch mindestens eines der folgenden Merkmale **gekennzeichnet sind:**
∘ mittlere Teilchengröße: im Bereich von 5 µm bis 60 µm (gemessen mit Laserdiffraktion; 23 °C),
∘ Schmelztemperatur: unter 1000 °C,
∘ Dichte: 2,0 bis 2,8 g/cm³.

5. Das Verfahren nach einem der vorstehenden Ansprüche, wobei die Zusammensetzung zusätzlich mindestens einen oder mehrere der folgenden Bestandteile umfasst:
a. einen rheologischen Modifikator mit einem Molekulargewicht von über 500.000,
b. Polyethylenglycol mit einem Molekulargewicht von unter 10.000,
c. Verdickungsmittel, das sich von den rheologischen Modifikatoren unterscheidet, ausgewählt aus Stärke, Methylcellulose, Ethylcellulose, Hydroxylpropylcellulose, Carboxymethylcellulose, Kombinationen und Mischungen davon,
d. Pigmente oder Farbstoffe,
e. Konservierungsmittel
und Mischungen davon.

6. Das Verfahren nach einem der vorstehenden Ansprüche, wobei die Zusammensetzung durch eines oder mehrere der folgenden Merkmale **gekennzeichnet ist:**
∘ Viskosität der Zusammensetzung ohne Glas- und/oder Glaskeramikmaterial: im Bereich von 10 bis 1.000 mPas, gemessen bei einer Temperatur von 23 °C mit einer Scherrate s d(gamma)/dt von 50 s⁻¹,
∘ Gehalt an organischen Bestandteilen: unter 7 Gew.-%, bezogen auf das Gewicht der gesamten Zusammensetzung.

7. Das Verfahren nach einem der vorstehenden Ansprüche, wobei die Zusammensetzung die Bestandteile in den folgenden Mengen umfasst:
a. Flüssigkeit: von 15 Gew.-% bis 60 Gew.-%,
b. Glas- und/oder Glaskeramikmaterial: von 40 Gew.-% bis 85 Gew.-%,
c. vernetzbarer Bindemittelvorläufer: von 0,1 Gew.-% bis 10 Gew.-%,
d. rheologischer Modifikator: von 0 Gew.-% bis 4 Gew.-%,
e. Polyethylenglycol: von 0 Gew.-% bis 10 Gew.-%,
f. Verdickungsmittel: von 0 Gew.-% bis 4 Gew.-%,
g. Pigmente oder Farbstoffe: von 0 Gew.-% bis 2 Gew.-%,
h. Konservierungsmittel: von 0 Gew.-% bis 2 Gew.-%,
wobei sich Gew.-% auf das Gewicht der gesamten Zusammensetzung bezieht.

8. Das Verfahren nach einem der vorstehenden Ansprüche, wobei der Zahnverblendungsvorläufer durch eines oder mehrere der folgenden Merkmale **gekennzeichnet ist:**
• der Verblendungsvorläufer umfasst ein offenzelliges Material,
• der Verblendungsvorläufer umfasst ein Glas- und/oder Glaskeramikmaterial,
• der Verblendungsvorläufer ist in Bezug auf eine gesinterte Verblendung mit einem Vergrößerungsfaktor von zwischen 1,12 bis 1,9 proportional vergrößert,
• Wärmeausdehnungskoeffizient: 8 ^{∗} 10⁻⁶ K⁻¹ bis 15,8 ^{∗} 10⁻⁶K⁻¹,
• wobei die Materialdichte des Verblendungsvorläufers in einem Bereich von 0,6 g/cm³ bis 2,5 g/cm³ liegt.

9. Das Verfahren nach Anspruch 8, wobei die Zahnträgerstruktur durch eines oder mehrere der folgenden Merkmale **gekennzeichnet ist:**
• die Zahnträgerstruktur umfasst ein Keramikmaterial oder Metall,
• Wärmeausdehnungskoeffizient: 8 ^{∗} 10⁻⁶ K⁻¹ bis 15,8 ^{∗} 10⁻⁶K⁻¹,
• die Materialdichte der Zahnträgerstruktur liegt in einem Bereich von 5 g/cm³ bis 19 g/cm³.

10. Das Verfahren nach Anspruch 8, umfassend die folgenden Schritte:
• Bereitstellen eines Zahnverblendungsvorläufers und einer Zahnträgerstruktur, wobei beide eine obere und eine untere Oberfläche aufweisen,
• Befestigen des Zahnverblendungsvorläufers mit seiner unteren Oberfläche an der oberen Oberfläche der Zahnträgerstruktur, umfassend den Schritt des Platzierens der Zusammensetzung wie in einem der Ansprüche 1 bis 8 beschrieben zwischen der unteren Oberfläche des Zahnverblendungsvorläufers und der oberen Oberfläche der Zahnträgerstruktur
• und gegebenenfalls einen Erhitzungsschritt, wobei der Erhitzungsschritt durch eines oder mehrere der folgenden Merkmale **gekennzeichnet ist:**
- Temperatur: zwischen 500 °C bis 1200 °C,
- Dauer: zwischen 1 min bis 1 h,
- atmosphärischer Druck: zwischen 25 und 1025 mbar.

11. Verwendung einer Zusammensetzung, die in dem Behälter wie in einem der Ansprüche 1 bis 7 beschrieben enthalten ist, zum Verblenden, Modellieren, Formen oder Anpassen eines Dentalartikels, wobei die Zusammensetzung Glas- und/oder Glaskeramikteilchen umfasst, die in einem Gel enthalten sind, wobei das Gel durch eine Sol/Gel-Reaktion eines Si und O erhältlich ist und anorganischen vernetzbaren Bindemittelvorläufer enthält, der in einer Flüssigkeit enthalten ist, wobei der vernetzbare Bindemittelvorläufer der Zusammensetzung aus Orthosilikaten, Kieselsol, Wasserglas, Mischungen davon ausgewählt ist und wobei der Dentalartikel aus einer Zahnträgerstruktur, einem Zahnverblendungsvorläufer, einem Abutment oder einem Dentalimplantat ausgewählt ist.

12. Kit aus Teilen, umfassend mindestens zwei Behälter wie in einem der Ansprüche 1 bis 7 beschrieben, wobei mindestens eine der in dem Behälter enthaltenen Zusammensetzungen zusätzlich einen Farbstoff oder ein Pigment umfasst, wobei die Zusammensetzung Glas- und/oder Glaskeramikteilchen umfasst, die in einem Gel enthalten sind, wobei das Gel durch eine Sol/Gel-Reaktion eines Si und O erhältlich ist und anorganischen vernetzbaren Bindemittelvorläufer enthält, der in einer Flüssigkeit enthalten ist, wobei der vernetzbare Bindemittelvorläufer der Zusammensetzung aus Orthosilikaten, Kieselsol, Wasserglas und Mischungen davon ausgewählt ist.

## Revendications

1. Procédé ex vivo pour la production d'une restauration dentaire ou une partie de celle-ci qui comprend les étapes consistant à
a. fournir un récipient contenant une composition à appliquer sur un article dentaire, la composition comprenant des particules de verre et/ou vitrocéramiques contenues dans un gel, le gel pouvant être obtenu par une réaction sol/gel d'un précurseur de liant réticulable inorganique contenant Si et O contenu dans un liquide, le récipient étant scellable ou scellé,
b. appliquer la composition contenue dans le récipient sur la surface d'un article dentaire,
dans lequel l'article dentaire ou une partie de celui-ci est une structure de support dentaire, un précurseur de facette dentaire, une butée ou un implant dentaire et
dans lequel le précurseur de liant réticulable de la composition est choisi parmi des orthosilicates, un sol de silice, du verre soluble, et des mélanges de ceux-ci.

2. Procédé selon la revendication 1, dans lequel le précurseur de liant réticulable de la composition est **caractérisé par** au moins l'une des caractéristiques suivantes :
∘ liquide dans des conditions ambiantes ou applicable en tant que solution ou dispersion aqueuse,
∘ densité : de 0,7 à 1,5 g/cm³
∘ masse moléculaire : au moins 100 g/mol, et/ou
∘ produit seulement une condensation à bas point d'ébullition ou des produits dérivés au cours d'une réaction de réticulation.

3. Procédé selon l'une quelconque des revendications précédentes, dans lequel la composition est **caractérisée par** une valeur de pH (composition aqueuse) étant dans la plage basique si le précurseur de liant réticulable comprend un orthosilicate ou étant dans la plage acide si le précurseur de liant réticulable comprend du verre soluble, un sol de silice, ou des mélanges de ceux-ci.

4. Procédé selon l'une quelconque des revendications précédentes, dans lequel les particules de verre ou vitrocéramiques dans la composition sont **caractérisées par** au moins l'une des caractéristiques suivantes :
∘ taille de particule moyenne : plage allant de 5 µm à 60 µm, (mesurée par diffraction laser ; 23 °C),
∘ température de fusion : inférieure à 1000 °C,
∘ densité : 2,0 à 2,8 g/cm³.

5. Procédé selon l'une quelconque des revendications précédentes, dans lequel la composition comprend en outre au moins un ou plusieurs des composants suivants :
a. un modificateur rhéologique avec un poids moléculaire supérieur à 500 000,
b. du polyéthylène glycol avec un poids moléculaire inférieur à 10 000,
c. un agent épaississant étant différent des modificateurs rhéologiques choisi parmi l'amidon, la méthylcellulose, l'éthylcellulose, l'hydroxypropylcellulose, la carboxyméthylcellulose, des combinaisons et des mélanges de ceux-ci,
d. des pigments ou des colorants,
e. un agent conservateur,
et des mélanges de ceux-ci.

6. Procédé selon l'une quelconque des revendications précédentes, dans lequel la composition est **caractérisée par** une ou plusieurs des caractéristiques suivantes :
∘ viscosité de la composition sans matériau de verre et/ou vitrocéramique : étant dans la plage allant de 10 à 1000 mPas, mesurée à une température de 23 °C avec une vitesse de cisaillement s d(gamma)/dt de 50 s⁻¹,
∘ teneur en composants organiques : étant en dessous de 7 % en poids par rapport au poids de l'ensemble de la composition.

7. Procédé selon l'une quelconque des revendications précédentes, dans lequel la composition comprend les composants dans les quantités suivantes :
a. liquide : de 15 % en poids à 60 % en poids,
b. matériau de verre et/ou vitrocéramique : de 40 % en poids à 85 % en poids,
c. précurseur de liant réticulable : de 0,1 % en poids à 10 % en poids,
d. modificateur rhéologique : de 0 % en poids à 4 % en poids,
e. polyéthylène glycol : de 0 % en poids à 10 % en poids,
f. agent épaississant : de 0 % en poids à 4 % en poids,
g. pigments ou colorants de 0 % en poids à 2 % en poids,
h. agent de conservation : de 0 % en poids à 2 % en poids,
% en poids par rapport au poids de la composition totale.

8. Procédé selon l'une quelconque des revendications précédentes, dans lequel le précurseur de facette dentaire est **caractérisé par** une ou plusieurs des caractéristiques suivantes :
• le précurseur de facette comprenant un matériau à alvéoles ouvertes,
• le précurseur de facette comprenant un matériau de verre et/ou vitrocéramique,
• le précurseur de facette étant proportionnellement agrandi par rapport à une facette frittée selon un facteur de grossissement compris entre 1,12 et 1,9,
• coefficient de dilatation thermique : allant de 8 ^{∗} 10⁻⁶ K⁻¹ à 15,8 ^{∗} 10⁻⁶ K⁻¹,
• la densité de matériau du précurseur de facette étant dans une plage allant de 0,6 g/cm³ à 2,5 g/cm³.

9. Procédé selon la revendication 8, dans lequel la structure de support dentaire est **caractérisée par** une ou plusieurs des caractéristiques suivantes :
• la structure de support dentaire comprenant un matériau céramique ou en métal,
• coefficient de dilatation thermique : allant de 8 ^{∗} 10⁻⁶ K⁻¹ à 15,8 ^{∗} 10⁻⁶ K⁻¹,
• la densité de matériau de la structure de support dentaire étant dans une plage allant de 5 g/cm³ à 19 g/cm³.

10. Procédé selon la revendication 8 comprenant les étapes suivantes :
• fournir un précurseur de facette dentaire et une structure de support dentaire, ayant l'un et l'autre une surface supérieure et une surface inférieure,
• fixer le précurseur de facette dentaire avec sa surface inférieure à la surface supérieure de la structure de support dentaire, comprenant l'étape de mise en place de la composition telle que décrite dans l'une quelconque des revendications 1 à 8 entre la surface inférieure du précurseur de facette dentaire et la surface supérieure de la structure de support dentaire
• et facultativement une étape de chauffage, dans laquelle l'étape de chauffage est **caractérisée par** une ou plusieurs des caractéristiques suivantes :
- température : entre 500 °C et 1200 °C,
- durée : entre 1 min et 1 h,
- pression atmosphérique : entre 25 et 1025 mbar.

11. Utilisation d'une composition contenue dans le récipient tel que décrit dans l'une quelconque des revendications 1 à 7 pour le plaquage, le modelage, la formation ou l'adaptation d'un article dentaire, la composition comprenant des particules de verre et/ou vitrocéramiques contenues dans un gel, le gel pouvant être obtenu par une réaction sol/gel d'un précurseur de liant réticulable inorganique contenant Si et O contenu dans un liquide, dans laquelle le précurseur de liant réticulable de la composition est choisi parmi des orthosilicates, un sol de silice, du verre soluble, des mélanges de ceux-ci, et dans laquelle l'article dentaire est choisi parmi une structure de support dentaire, un précurseur de facette dentaire, une butée ou un implant dentaire.

12. Kit de pièces comprenant au moins deux récipients tels que décrits dans l'une quelconque des revendications 1 à 7, dans lequel au moins une des compositions contenues dans le récipient comprend en outre un colorant ou un pigment, la composition comprenant des particules de verre et/ou vitrocéramiques contenues dans un gel, le gel pouvant être obtenu par une réaction sol/gel d'un précurseur de liant réticulable inorganique contenant Si et O contenu dans un liquide, dans lequel le précurseur de liant réticulable de la composition est choisi parmi des orthosilicates, un sol de silice, du verre soluble, et des mélanges de ceux-ci.
